# EUROPEAN PATENT APPLICATION

(11) **EP 3 415 099 A1**
(43) Date of publication of application: **19.12.2018**
(21) Application number: 17176474.9
(22) Date of filing: 16.06.2017
(51) Int. Cl.: A61B 10/02

(54) **MEDICAL MUILTIPLE SKIN BIOPSY DEVICE**

(71) Applicant: Galderma S.A., 6330 Cham (CH)
(72) Inventor: WEILER, Thibaud, 06270 Villeneuve Loubet (FR); MAURY, Ludivine, 06600 Antibes (FR)
(74) Representative: AWA Sweden AB

(57) **Abstract**

A medical multiple skin biopsy device for removal of skin tissue comprising a body having a proximal end and a distal end, the distal end being arranged to engage with the skin surface; a tubular outer core element arranged inside the body and comprising a cutting portion at a distal end thereof; a tubular inner core element arranged inside the outer core element and comprising a cutting portion at a distal end thereof; and an operation unit connected with the outer and inner core elements.

A space is defined between at least a distal end portion of an outer wall of the inner core element and at least a distal end portion of an inner wall of the outer core element. The outer and inner core elements are axially moveable between a retracted position, and an extended position in which the cutting portions of the core elements protrude from the body. The operation unit is arranged for moving the core elements between the retracted and extended positions, whereby the medical skin biopsy device is arranged for cutting at least two skin tissue samples in one operation.

## Description

### FIELD OF TECHNOLOGY

The present invention relates to a medical multiple skin biopsy device for removal of skin tissue, the device having a body with a proximal end and a distal end, the distal end being arranged to engage with the skin surface.

### BACKGROUND OF THE INVENTION

A skin biopsy is a well-known medical procedure for diagnosing skin disorders or to be used as a research tool on skin samples. When performed on living beings, the usual procedure is to anesthetize the biopsy site, excise a small cylindrical specimen for analysis at a pathology laboratory and to generally repair the biopsy site with sutures. Thus, skin biopsy is an invasive procedure which can be painful for patient. The main disadvantage of this procedure is that it requires the use of several invasive gestures. Generally, a maximum of three skin tissue samples can be removed from one patient, wherein the removal involves the use of local anesthetic, and an invasive procedure for each skin biopsy. One example of a device for performing skin biopsies is disclosed in EP 3081170, where a tube with a sharp end is driven into the skin during rotation. During the design of clinical studies, the number of skin biopsies performed in a patient is a limiting factor for the study in terms of cost for the pharmaceutical company and in terms of ethics for the patient. There is consequently a need to limit invasive acts related to biopsies sampling on human or animals. When used as a research tool, the multiple biopsies as performed today show many drawbacks. The size of available skin samples may not allow to obtain a sufficient number of biopsies to perform the requested analysis. Another issue faced is that performing a first biopsy on a skin sample weaken the remaining part of the skin, leading to difficulties for obtaining additional valid biopsies from the same sample. As a research tool, there is also a need to improve biopsy sampling.

### SUMMARY OF THE INVENTION

It would be advantageous to be able to increase the number of skin tissue samples from a patient without having to make more invasive acts as well as to improve the biopsies sampling for research purposes

To better address this concern, in a first aspect of the invention there is presented a medical skin biopsy device for removal of skin tissue comprising:
a body having a proximal end and a distal end, the distal end being arranged to engage with the skin surface; a tubular outer core element arranged inside the body, wherein the outer core element comprises a cutting portion at a distal end thereof; a tubular inner core element arranged inside the outer core element and comprising a cutting portion at a distal end thereof; and an operation unit connected with the outer and inner core elements. A space is defined between at least a distal end portion of an outer wall of the inner core element and at least a distal end portion of an inner wall of the outer core element. The outer and inner core elements are axially moveable between a retracted position, in which retracted position the distal ends of the respective outer and inner core elements are positioned inside the body, and an extended position, in which extended position the distal ends of the respective outer and inner core elements are positioned such that they protrude from the distal end of the body. The operation unit is arranged for moving the outer and inner core elements between the retracted and extended positions, whereby the medical skin biopsy device is arranged for cutting at least two skin tissue samples in one operation. Thereby, the skin biopsy device comprises a mechanism of multiple axially movable tubular core elements, by means of which it is possible to perform multiple skin biopsies for removal of multiple, and preferably two, skin tissue samples from the skin in one operation, i.e. in a single invasive act. Thus, the device can be considered to be a medical multiple skin biopsy device. Fetching multiple skin tissue samples in one single invasive act could minimize the patient pain and increase the total number of data collected.

In accordance with embodiments of the skin biopsy device, the operation unit is arranged to drive either the inner core element or both the inner and the outer core elements to rotate about a longitudinal axis thereof, at least during movement of the outer and inner core elements out of the body to the extended position. Thereby, the entrance into the skin is facilitated.

In accordance with an embodiment of the skin biopsy device, the outer and inner core elements are coaxially arranged. They can be non-coaxially arranged as well, but the coaxial arrangement provides for a simpler structure of the drive unit.

In accordance with an embodiment of the skin biopsy device, the operation unit is arranged to drive the outer and inner core elements in opposing rotational movements. This counteraction facilitates a simultaneous movement of the outer and inner core elements into the skin tissue.

In accordance with an embodiment of the skin biopsy device, the operation unit comprises a piston extending into the inner core element from its proximal end, wherein the piston is longitudinally movable relative to the inner core element, and is in threaded engagement therewith for causing the inner core to rotate during the longitudinal movement of the piston. Thereby...

In accordance with an embodiment of the skin biopsy device, it comprises a cutting mechanism arranged to cut the skin tissue enclosed by the outer core element in the extended position. Thereby, it is ensured that the tissue samples are fully removed and brought along with the skin biopsy device when it is removed after having been operated.

In accordance with an embodiment of the skin biopsy device, it comprises an ejector unit arranged to eject the skin tissue samples from the medical skin biopsy device after having performed the biopsy. For embodiments where the tissue samples are fully removed from the patient by means of the present device, the ejector unit facilitates the following examination of the tissue samples.

### BRIEF DESCRIPTION OF THE DRAWINGS

The invention will now be described in more detail and with reference to the appended drawings in which:
Fig. 1 is a schematic cross-sectional view showing an embodiment of the skin biopsy device according the present invention;
Figs. 2 and 3 are schematic perspective views showing embodiments of the skin biopsy device according to the present invention;
Fig. 4 is a schematic cross-sectional view showing an embodiment of the skin biopsy device according the present invention;
Fig. 5 is a schematic perspective view showing an embodiment of the skin biopsy device according to the present invention; and
Fig. 6 is a schematic cross-sectional view showing a portion of an embodiment of the skin biopsy device according the present invention.

### DESCRIPTION OF EMBODIMENTS

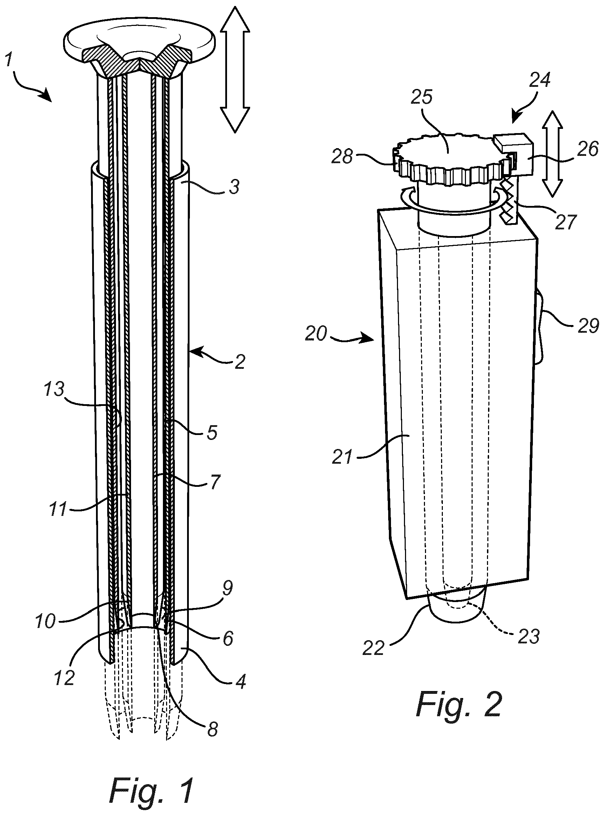

According to a first embodiment of the medical skin biopsy device 1, the operational principle of which is shown most schematic in Fig. 1, it comprises a body 2 having a proximal end 3 and a distal end 4, the distal end 4 being arranged to engage with the skin surface, a tubular outer core element 5 arranged inside the body 2, comprising a cutting portion 6 at its distal end, and a tubular inner core element 7 arranged inside the outer core element and comprising a cutting portion 8 at its distal end. In this embodiment, the body 2 is tubular as well. The outer and inner core elements 5, 7 are coaxially arranged at a radial distance from each other. At least there is a space 9 defined between at least a distal end portion 10 of an outer wall 11 of the inner core element 7, and at least a distal end portion 12 of an inner wall 13 of the outer core element 5. This space 9 allows for generating a ring shaped tissue sample, while generating a circle shaped sample within the inner core element 7.

The outer and inner core elements 5, 7 are axially moveable between a retracted position, in which retracted position the distal ends of the respective outer and inner core elements 5, 7 are positioned inside the body 2, as shown in Fig. 1, and an extended position, in which extended position the distal ends of the respective outer and inner core elements 5, 7 are positioned such that they protrude from the distal end 4 of the body 2, as shown by means of broken lines in Fig. 1. The movement between the retracted and extended positions is performed by means of an operation unit 14, which in this embodiment simply is a top portion attached to the outer and inner core elements 5, 7 at their distal ends.

In accordance with a second embodiment of the skin biopsy device 20, it has the same basic parts comprised of a body 21, an outer core element 22, an inner core element 23, and an operation unit 24. However, in this second embodiment, in addition to being longitudinally moveable relative to the body 21, the outer and inner core elements 22, 23 are rotational, and the operation unit 24 is an electronic device comprising a top portion 25 attached to the outer and inner core elements 22, 23, and a drive element 26 mounted on a drive shaft 27, which is longitudinally movable. The drive element is engaged with the top portion 25, which is provided with peripheral cogs 28. The operation unit 24 further comprises a control unit, mounted internally of the body 21, and not shown in the drawings, and a switch 29 connected with the control unit. By operating the switch 29, the control device is activated to move the drive shaft 27 and to drive the drive element 26 in order to extend or retract the outer and inner core elements 22, 23. At least during the extension the outer and inner core elements 22, 23 are rotated. The rotation facilitates the intrusion into the skin tissue.

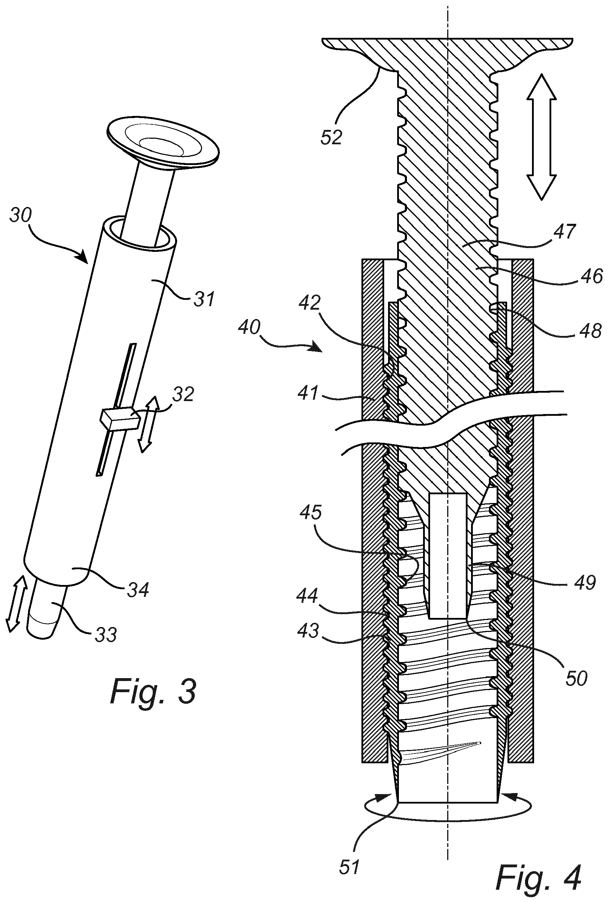

In accordance with a third embodiment of the skin biopsy device 30, shown in Fig. 3, it comprises a body 31, and an ejector member 32, which is arranged to eject the skin tissue samples collected by means of the outer and inner core elements 33. The ejector member 32 comprises internal push portions, extending into the inner core element and into the space between the inner and outer core elements. The push portions are arranged to push the skin tissue samples out of the distal ends of the outer and inner core elements 33 when the ejector member 32 is moved towards the distal end 34 of the body 31. Alternatively, the ejector member can be provided as a fixed member, is positioned close to the distal end of the body, and which stops the skin tissue samples from being brought along with the core elements into the body.

In accordance with a fourth embodiment of the skin biopsy device 40, as shown in Fig. 4, the body 41 is provide with a first thread 42 at an inner tubular surface thereof. The outer core element 43 is provided with a second thread 44 at an outer surface thereof, and a third thread 45 at an inner surface thereof. The first and second threads 42, 44 are engaged with each other. The inner core element 46 has a proximal portion 47 which is provided with a fourth thread 48 at an outer surface thereof, which is engaged with the third thread 45, and a distal portion 49, which includes the sharp distal end 50 and is radially spaced from the outer core element 43. In the retracted position, the distal end 50 of the inner core element 46 is further retracted into the body 41 then the distal end 51 of the outer core element 43. The third and fourth threads have a steeper pitch than the first and second threads 42, 44. The inner core element 46 is longitudinally movable but not rotatable, while the outer core element 43 is both longitudinally movable, i.e. displaceable, and rotatable. Thus, when operating the operation unit 52, arranged at the inner core element 46, to extend the inner and outer core elements 46, 43 out of the body 41, the distal end 50 of the inner core element 46 will reach as far out of the body 41 as the distal end 51 of the outer core element 43.

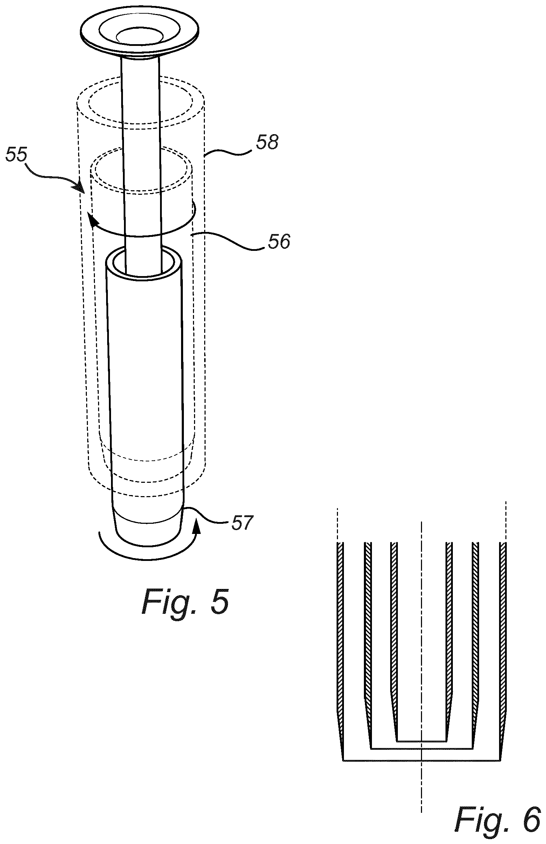

In accordance with a fifth embodiment of the skin biopsy device 55, shown in Fig. 5, the inner core element 57 and the outer core element 56 are arranged to rotate in opposite rotational directions while being extended out of the body 58. This counter rotation further facilitates the entrance into the skin tissue of the outer and inner cores 56, 57.

A sixth embodiment of the skin biopsy device 60 comprises three core elements 61, 62, 63, being coaxially arranged, thereby generating three tissue samples in one invasive act.

The diameter of the core elements is an interesting factor, since it is generally not necessary to end the skin biopsy procedure with sutures if the skin tissue sample is no bigger than about 2 mm in diameter. Therefore, in all embodiments, a preferred maximum inner diameter of the outer core element is 2 mm. By avoiding suturing, the efficiency of biopsies is increased and time is saved for the physician and the patient.

However, in some applications, such as for example when the skin biopsy device is used as a research tool on skin samples, larger skin tissue samples of up to 3-6 mm can be removed. It should be mentioned that the skin biopsy device can be used for human or veterinary medicine, and for biopsies on living patients as well as on separate skin portions, which have been removed during surgery or the like. In the latter case, there is no problem of pain or ethics, which makes it possible to remove larger skin tissue samples. The present device is however still advantageous in that it saves time, limits the number of acts, improve the quality of biopsy sample by performing the sampling in once, etc.

In general, the use of the skin biopsy device for taking multiple skin tissue samples in a single invasive act could reduce the use of anesthetic, the total number of invasive acts for a patient, and provide for minimal scars in the skin of the patient.

As understood from the above description the embodiments of the skin biopsy device, the single invasive act to fetch multiple, preferably two, skin tissue samples, involves, in a first step, positioning the skin biopsy device at the skin so that its distal end is engaged with the skin surface, and, in a second step, operating the operation unit to drive the core elements into the skin tissue to a predetermined sample depth. Under certain circumstances, the skin tissue samples are completely loosed during the second step, while it typically is necessary to perform a third step, in which the skin tissue is cut at the sample depth by means of a separate pair of scissors. However, in alternative embodiments the skin biopsy device comprises a cutting mechanism, which is either individually operated by means of a cut operation element, or which is automatically activated at the end of the extension of the core elements. The cutting mechanism can be any appropriate type of mechanism, for instance similar to a cigar cutter having a sharp blade movable across the openings of the core elements.

Further mechanical solutions for the operational movement of the multiple biopsy device are feasible, where the movement mechanism disclosed in EP3081170 modified to operate the multiple core elements is one example.

While the invention has been illustrated and described in detail in the drawings and foregoing description, such illustration and description are to be considered illustrative or exemplary and not restrictive. The invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. Any reference signs in the claims should not be construed as limiting the scope.

## Claims

1. A medical skin biopsy device for removal of skin tissue comprising:
a body having a proximal end and a distal end, the distal end being arranged to engage with the skin surface;
a tubular outer core element arranged inside the body and comprising a cutting portion at a distal end thereof;
a tubular inner core element arranged inside the outer core element and comprising a cutting portion at a distal end thereof; and
an operation unit connected with the outer and inner core elements;
wherein a space is defined between at least a distal end portion of an outer wall of the inner core element and at least a distal end portion of an inner wall of the outer core element,
wherein the outer and inner core elements are axially moveable between a retracted position, in which retracted position the distal ends of the respective outer and inner core elements are positioned inside the body, and an extended position, in which extended position the distal ends of the respective outer and inner core elements are positioned such that they protrude from the distal end of the body; and
wherein the operation unit is arranged for moving the outer and inner core elements between the retracted and extended positions,
whereby the medical skin biopsy device is arranged for cutting at least two skin tissue samples in one operation.

2. The medical skin biopsy device according to claim 1, wherein the drive unit is arranged to drive the inner core element to rotate about a longitudinal axis thereof, at least during movement of the outer and inner core elements out of the body to the extended position.

3. The medical skin biopsy device according to claim 2, wherein the drive unit is additionally arranged to drive the outer core element to rotate about a longitudinal axis thereof, at least during the movement of the outer and inner core elements out of the body to the extended position.

4. The medical skin biopsy device according to claim 3, wherein the outer and inner core elements are coaxially arranged.

5. The medical skin biopsy device according to claim 3 or 4, wherein the drive unit is arranged to drive the outer and inner core elements in opposing rotational movements.

6. The medical skin biopsy device according to any one of claims 2 to 5, wherein the drive unit comprises a piston extending into the inner core element from its proximal end, wherein the piston is longitudinally movable relative to the inner core element, and is in threaded engagement therewith for causing the inner core to rotate during the longitudinal movement of the piston.

7. The medical skin biopsy device according to any one of the preceding claims, comprising a cutting mechanism arranged to cut the skin tissue enclosed by the outer core element in the extended position.

8. The medical skin biopsy device according to any one of the preceding claims, comprising an ejector unit arranged to eject the skin tissue samples from the medical skin biopsy device after having performed the invasive act.

9. The medical skin biopsy device according to any one of the preceding claims, wherein an inner diameter of the outer core element is at most 6 millimetres.

10. The medical skin biopsy device according to any one of the preceding claims, wherein an inner diameter of the outer core element is at most 2 millimetres.
